(19) Europäisches Patentamt
European Patent Office
Office européen des brevets

(11) **EP 4 521 077 A1**

(12) **EUROPEAN PATENT APPLICATION**

(43) Date of publication:
**12.03.2025 Bulletin 2025/11**

(21) Application number: **23196250.7**

(22) Date of filing: **08.09.2023**

(51) International Patent Classification (IPC):
**G01F 23/284** $^{(2006.01)}$     **G01S 13/88** $^{(2006.01)}$

(52) Cooperative Patent Classification (CPC):
**G01F 23/284; G01S 13/88**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Rosemount Tank Radar AB**
**435 23 Mölnlycke (SE)**

(72) Inventors:
• **FRIEDMANN, Anders**
  **582 46 LINKÖPING (SE)**
• **DELIN, Håkan**
  **585 93 LINKÖPING (SE)**
• **GRAHN, Marcus**
  **582 76 LINKÖPING (SE)**
• **ERIKSSON, Mikael**
  **593 52 VÄSTERVIK (SE)**

(74) Representative: **Kransell & Wennborg KB**
**P.O. Box 2096**
**403 12 Göteborg (SE)**

(54) **TANK ARRANGEMENT AND METHOD FOR ESTIMATING A SIGNAL PROPAGATION PROPERTY OF A SUBSTANCE COMPOSITION IN A PREDEFINED TANK PORTION**

(57)     A tank arrangement, comprising a tank having a first tank portion and a second tank portion above the first tank portion; an inlet for receiving a substance composition; at least a first radar gauging system arranged at the tank, the first radar gauging system comprising: a first transceiver; a first transmission line probe having an impedance discontinuity spaced apart along the first transmission line probe from the first probe end by a known first distance, the first transmission line probe being arranged with a portion of the first transmission line probe extending from the first probe end to the impedance discontinuity exclusively located in the first tank portion; and first processing circuitry coupled to the first transceiver and configured to estimate a signal propagation property of the substance composition within a first volume surrounding the portion of the first transmission line probe.

Fig. 1

## Description

Technical Field of the Invention

The present invention relates to a tank arrangement, and to a method of estimating a signal propagation property of a substance composition in a tank.

Technical Background

In different industrial applications, there is a need to reliably separate different substances with different densities. As an illustrative example, the petroleum industry uses so-called phase separators for separating water, oil, and gas. The separated water, oil, and gas are output through different outlets for recycling, discharge or further refinement, for example. In this and other applications, it is desirable to achieve distinct separations, for example avoiding output of an emulsion of water and oil through either of the outlets intended for output of water or oil. There is thus a need for enabling an estimation of a material composition in a predefined portion of a tank, such as close to a bottom of the tank.

Summary of the Invention

In view of the above, it would be desirable to provide for improved estimation of a signal propagation property of a substance composition within a predefined volume in a tank. In particular, the predefined volume may be near a bottom of a tank.

According to a first aspect of the present invention, it is therefore provided a tank arrangement, comprising: a tank having a first tank portion defined by a first range of vertical distances from a bottom of the tank, and a second tank portion defined by a second range of vertical distances from the bottom of the tank, the second range of vertical distances only containing distances greater than all distances in the first range of vertical distances; an inlet for receiving a substance composition including at least a liquid first substance having a first density and a liquid second substance having a second density lower than the first density; at least a first radar gauging system arranged at the tank, the first radar gauging system comprising: a first transceiver for generating, transmitting and receiving electromagnetic signals; a first transmission line probe having a first probe end coupled to the first transceiver and an impedance discontinuity spaced apart along the first transmission line probe from the first probe end by a known first distance, the first transmission line probe being arranged inside the tank in such a way that a portion of the first transmission line probe extending from the first probe end to the impedance discontinuity is exclusively located in the first tank portion, the first transmission line probe being configured to guide an electromagnetic transmit signal from the first transceiver from the first probe end to the impedance discontinuity, and to return to the first transceiver an electromagnetic reflection signal resulting from reflection of the transmit signal at the impedance discontinuity; and first processing circuitry coupled to the first transceiver and configured to estimate a signal propagation property of the substance composition within a first volume surrounding the portion of the first transmission line probe, based on a timing relation between the transmit signal and the reflection signal, and on the known first distance.

The present invention is based upon the realization that a material composition within a predefined level range in a tank can be estimated using an estimation of a signal propagation property in that level range. The present inventors have further realized that such an estimation of a signal propagation property can be achieved using a transmission line probe with a probe portion that is located exclusively within the predefined level range (corresponding to a tank portion) if the length along the transmission line probe of the probe portion is known, and can also be measured based on a relation between an electromagnetic signal guided along the probe portion from a transceiver and back following reflection at the far end of the probe portion. To enable this measurement, the inventors have found that it is beneficial if the probe portion starts with a first probe end that is connected to the transceiver. This provides for a configuration where the electromagnetic signal is not guided through the substance composition in another level range before reaching the predefined level range, which is spaced apart from a ceiling of the tank.

Hereby, reliable and accurate estimation of a material composition in a predefined first tank portion, such as close to a bottom of the tank, can be achieved, whereby improved control of the tank arrangement can be provided for.

The tank arrangement may comprise an outlet arranged in the first tank portion for output of first substance separated from the substance composition. The output may be controlled based on the estimated signal propagation property of the substance composition within the first volume surrounding the portion of the first transmission line probe, in such a way that output of second substance through the outlet is prevented, or at least minimized. This may be desirable, in particular in applications where the first substance is water and the second substance is a hydrocarbon, such as oil.

In various embodiments of the tank arrangement according to the present invention, the first transmission line probe may comprise a probe conductor coupled to the transceiver; and a dielectric layer at least partly enclosing the probe conductor.

The dielectric layer may advantageously form a dielectric enclosing structure extending along a substantial portion of the transmission line probe, such as along the entire length of the transmission line probe arranged inside the tank.

This type of the transmission line probe can be referred to as a Partially External Dielectric (PED) transmission line probe.

The propagation velocity along a PED transmission line probe is characterized by an effective dielectric constant $\varepsilon_{eff}$ which depends on the dielectric constant of the dielectric enclosing structure $\varepsilon_{int}$ and the dielectric constant of the surrounding medium $\varepsilon_{ext}$. The propagation velocity of the electromagnetic signal (transmit signal and reflection signal) travelling along the PED transmission line probe is given by the speed of light divided by the square root of $\varepsilon_{eff}$.

The effective dielectric constant $\varepsilon_{eff}$ of the PED transmission line probe at least approximately depends on the dielectric constant of the dielectric enclosing structure $\varepsilon_{int}$ and the dielectric constant of the surrounding medium $\varepsilon_{ext}$ according to the following relation:

$$\varepsilon_{eff} \sim \frac{1}{\dfrac{\alpha}{\epsilon_{ext}} + \dfrac{1-\alpha}{\varepsilon_{int}}}$$

where $\alpha$ is a number between 0 and 1 which indicates the degree of coupling to the surrounding medium. With $\alpha$=1, the probe conductor is uncoated (thus maximum influence of the surrounding medium), and with $\alpha$=0, the probe conductor is fully screened from the surrounding medium (the signal propagation is completely independent of the surrounding medium).

In embodiments of the tank arrangement according to the present invention, the first transmission line probe may advantageously comprise a a substantially uniform dielectric material coating with a thickness and material properties selected to achieve a coupling coefficient $\alpha$ that is greater than 0.1 and less than 0.5, to achieve a suitable tradeoff between coupling with the surrounding substance composition and signal loss along the portion of the first transmission line probe. As an illustrative and non-limiting example, the dielectric material coating may be made of PTFE and a have a thickness in the range 1 mm to 10 mm.

In embodiments of the tank arrangement according to the invention, the impedance discontinuity of the first transmission line probe of the first radar gauging system may be at a higher vertical level than the first probe end. For example, the first transmission line probe may be substantially vertically arranged, with the first probe end at a bottom of the tank. In other example configurations, the first transmission line probe may be substantially horizontally arranged.

The impedance discontinuity of the first transmission line probe of the first radar gauging system may be constituted by a second probe end of the first transmission line probe. This may provide for a relatively compact and simple configuration of the first radar gauging system. As an alternative, the impedance discontinuity may be provided in the form of a structure along the probe providing an impedance discontinuity, such as a metallic reflector structure. Such a configuration may provide for more detailed information about the signal propagation property of the substance composition in sub-volumes of the first volume.

In various embodiments, the tank arrangement may be configured as a phase separator arrangement. In such embodiments, the tank may be elongated in a substantially horizontal first direction, and extending in the first direction between a first tank end and a second tank end. The inlet may be adjacent to the first tank end. The tank may comprise an overflow weir arranged at a weir position between the inlet and the second tank end. The tank may comprise a first outlet arranged at a bottom of the tank horizontally between the inlet and the weir position, for output of first substance separated from the substance composition, and a second outlet arranged at a bottom of the tank horizontally between the weir position and the second tank end, for output of second substance separated from the substance composition. The first radar gauging system may be arranged to gauge the substance composition at a first gauging position between the inlet and the weir position.

Using information obtainable from the first radar gauging system, operation of this phase separator arrangement can be optimized. For instance, a flow rate of the substance composition provided to the inlet can be controlled based on the estimation of the signal propagation property provided by the first radar gauging system. In particular, the estimation of the signal propagation property may indicate the efficiency of the separation of the substance composition into the first substance and the second substance. If the estimation of the signal propagation property indicates a separation that is below a predefined threshold separation, the flow rate of the substance composition through the inlet may be reduced or the flow of substance composition through the inlet may be temporarily stopped.

According to various embodiments, furthermore, the tank arrangement of the invention may comprise a second radar gauging system arranged at the tank. The second radar gauging system may include a second transceiver for generating, transmitting and receiving electromagnetic signals, a second transmission line probe having a first probe end coupled to the second transceiver, the second transmission line probe being configured to guide an electromagnetic transmit signal, provided by the second transceiver, from the first probe end towards the substance composition in the tank, and to return to the second transceiver an electromagnetic reflection signal resulting from reflection of the transmit signal at a surface of the substance composition; and second processing circuitry coupled to the second transceiver and configured to estimate, based on a timing relation between the transmit signal and the reflection signal, a level of the surface of the substance composition.

The provision of this second radar gauging system provides for improved process control for the tank arrangement.

The second transmission line probe may advantageously be vertically extending, to allow arrangement of an external measurement unit of the second radar gauging system on the top of the tank.

In various example configurations, the second transmission line probe may have an impedance discontinuity spaced apart along the second transmission line probe from the first probe end by a known second distance, the second transmission line probe being arranged inside the tank in such a way that a portion of the second transmission line probe extending from the first probe end to the impedance discontinuity is exclusively located in the second tank portion; and the second processing circuitry may be configured to estimate, based on the timing relation between the transmit signal and the reflection signal and on the known second distance, a signal propagation property of the substance composition within a second volume surrounding the portion of the second transmission line probe.

According to a second aspect of the present invention, it is provided a method of estimating a signal propagation property of a substance composition in a first tank portion defined by a first range of vertical distances from a bottom of the tank, the first tank portion being below a second tank portion defined by a second range of vertical distances from the bottom of the tank, the method comprising: providing a first transmission line probe having a first probe end and an impedance discontinuity spaced apart along the first transmission line probe from the first probe end by a known first distance, the first transmission line probe being arranged inside the tank in such a way that a portion of the first transmission line probe extending from the first probe end to the impedance discontinuity is exclusively located in the first tank portion; generating and transmitting, by a first transceiver, an electromagnetic transmit signal; providing the transmit signal to the first probe end of the first transmission line probe; receiving, by the first transceiver from the first transmission line probe, an electromagnetic reflection signal resulting from reflection of the transmit signal at the impedance discontinuity of the first transmission line probe; and estimating, by first processing circuitry coupled to the first transceiver, the signal propagation property of the substance composition within a first volume surrounding the portion of the first transmission line probe, based on a timing relation between the transmit signal and the reflection signal, and on the known first distance.

Further embodiments of, and effects obtained through this second aspect of the present invention are largely analogous to those described above for the first aspect of the invention.

In summary, the present invention thus relates to a tank arrangement, comprising a tank having a first tank portion and a second tank portion above the first tank portion; an inlet for receiving a substance composition; at least a first radar gauging system arranged at the tank, the first radar gauging system comprising: a first transceiver; a first transmission line probe having an impedance discontinuity spaced apart along the first transmission line probe from the first probe end by a known first distance, the first transmission line probe being arranged with a portion of the first transmission line probe extending from the first probe end to the impedance discontinuity exclusively located in the first tank portion; and first processing circuitry coupled to the first transceiver and configured to estimate a signal propagation property of the substance composition within a first volume surrounding the portion of the first transmission line probe.

Brief Description of the Drawings

These and other aspects of the present invention will now be described in more detail, with reference to the appended drawings showing example embodiments of the invention, wherein:

Fig 1 schematically illustrates a first example of a tank arrangement according to embodiments of the present invention;

Figs 2A-B schematically illustrates an example configuration of the first radar gauging system comprised in the tank arrangement in fig 1;

Fig 3 schematically illustrates a second example of a tank arrangement according to embodiments of the present invention;

Fig 4 schematically illustrates an example configuration of the second radar gauging system comprised in the tank arrangement in fig 3;

Fig 5 schematically illustrates a third example of a tank arrangement according to embodiments of the present invention, in the form of a phase separator arrangement;

Fig 6 is a flow-chart schematically illustrating an example of the method according to the present invention;

Fig 7 is an exemplary echo curve obtained using the first radar gauging system in figs 2A-B; and

Fig 8 is an exemplary echo curve obtained using the second radar gauging system in fig 4.

Detailed Description of an Example Embodiment of the Invention

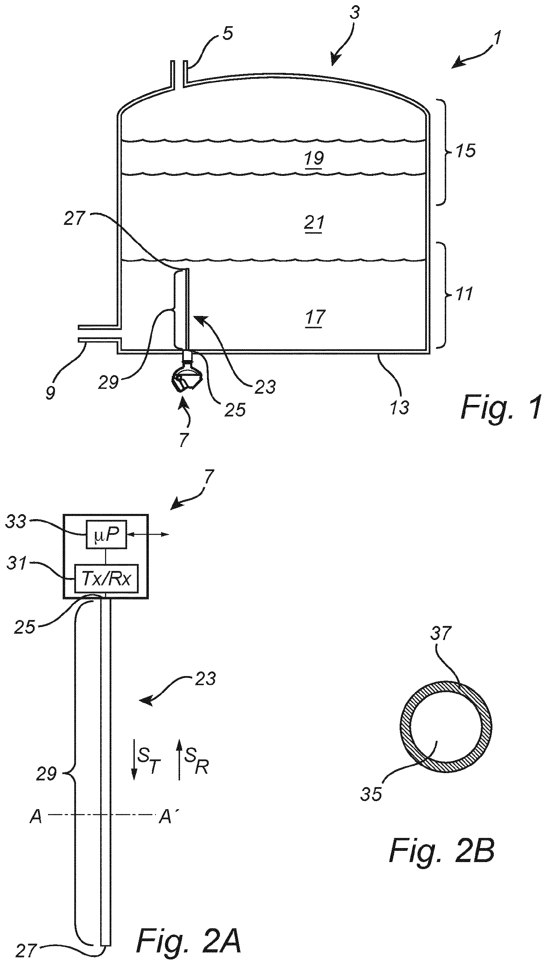

Fig 1 schematically illustrates a first example of a tank arrangement 1 according to embodiments of the present invention. The tank arrangement 1 according to the first example comprises a tank 3, an inlet 5, a first radar gauging system 7, and, optionally, an outlet 9. As is schematically indicated in fig 1, the tank 3 has a first tank portion 11, defined by a first range of vertical distances from a bottom 13 of the tank 3, and a second tank portion 15 defined by a second range of vertical distances from the bottom 13 of the tank 3. The first range and the second range are mutually non-overlapping.

When the tank arrangement 1 is in use, a flow of a substance composition is received through the inlet 5. The substance composition includes at least a liquid first substance 17 having a first density, and a liquid second substance 19 having a second density, lower than the first density. The first substance 17 may, for example, be water, and the second substance 19 may, for example, be oil. In the tank 3, the first substance 17 and the second substance 19 may be phase separated to a degree of separation that may, for example, depend on the settling time since the last input of substance composition through the inlet 5. There may, for example, be an emulsion layer 21 with unknown thickness between a bottom layer of pure first substance 17, and a top layer of pure second substance 19.

Because of the potentially time-varying and generally unknown degree of separation between the first substance 17 and the second substance 19, there is a need for estimating the substance composition in the first tank portion 11, for example to determine when and how to allow output through the outlet 9. For instance, the timing and/or the flow-rate of the output through the outlet 9 may be desirable to control based on an estimation of the substance composition in the first tank portion 11. In the illustrative case of the first substance 17 being water, and the second substance 19 being oil, it may be desirable to only output pure, or almost pure, water through the outlet 9, and not a water-oil emulsion.

In example embodiments of the present invention, the first radar gauging system 7 may be used to provide for the desired estimation of the substance composition in the first tank portion 11.

Referring to fig 1, the radar gauging system 7 comprises a first transmission line probe 23 having a first probe end 25 and an impedance discontinuity 27 spaced apart along the first transmission line probe 23 from the first probe end 25. The first transmission line probe 23 is arranged inside the tank 3 in such a way that a portion 29 of the first transmission line probe 23 extending from the first probe end 25 to the impedance discontinuity 27 is exclusively located in the first tank portion 11.

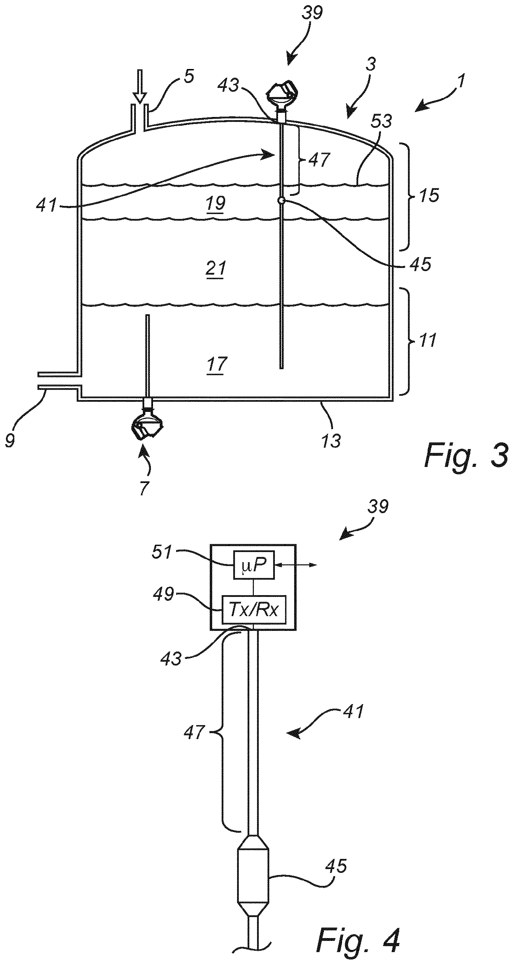

Figs 2A-B schematically illustrate an example configuration of the first radar gauging system 7 comprised in the tank arrangement in fig 1, where fig 2B schematically shows a cross-section of the first transmission line probe 23 in fig 4A, of a section taken along the line A-A' in fig 2A.

Referring first to fig 2A, the first radar gauging system 3 comprises the above-mentioned first transmission line probe 23, a first transceiver 31, and first processing circuitry 33. The first probe end 25 of the first transmission line probe 23 is coupled to the first transceiver 31, which is configured to generate and transmit an electromagnetic transmit signal $S_T$ to the first transmission line probe 23. The first transmission line probe 23 is configured to guide the transmit signal $S_T$ from the first probe end 25 to the impedance discontinuity 27 and to return to the first transceiver 31 an electromagnetic reflection signal $S_R$ resulting from reflection of the transmit signal

$S_T$ at the impedance discontinuity 27.

By evaluating a timing relation between the transmit signal $S_T$ and the reflection signal $S_R$, using techniques that are *per* se well-known to the person of ordinary skill in the art, the first processing circuitry 33 may be configured to estimate the distance from the first probe end 25, which is a reference location at an interface between the first transceiver 31 and the transmission line probe 23 and the impedance discontinuitiy 27. By comparing a distance thus estimated based on a time-of-flight of the transmit signal $S_T$ (and reflection signal $S_R$) along the portion 29 of the first transmission line probe 23 with the corresponding known first distance between the first probe end 25 and the impedance discontinuity, the propagation speed of the transmit signal $S_T$ (and reflection signal $S_R$) can be estimated. From the estimated propagation speed another signal propagation property, such as the (average) dielectric constant, can be estimated, and/or conclusions can be drawn about the material composition through which the transmit signal $S_T$ (and reflection signal $S_R$) has been propagated. The impedance discontinuity 27, may, for example, be provided by a second probe end as is schematically indicated in fig 2A, or by a reference impedance discontinuity spaced apart from the second probe end.

In the example configuration shown in figs 2A-B, the first transmission line probe 23 comprises a probe conductor 35 coupled to the first transceiver 31, and a dielectric layer 37 enclosing the probe conductor 35. The dielectric layer 37 may, for example, be made of PTFE and may have a thickness in the range of 1 mm to 10 mm.

Fig 3 schematically illustrates a second example of a tank arrangement 1 according to embodiments of the present invention, which tank arrangement mainly differs from that described above with reference to fig 1 in that it comprises a second radar gauging system 39 arranged at the tank 3. Referring to fig 3, the second radar gauging system 39 comprises a second transmission line probe 41, having a first probe end 43. In the example configuration schematically shown in fig 3, the second transmission line probe 41 additionally has a reference reflector 45 for providing an impedance discontinuity. As is indicated in fig 3, the second transmission line probe 41 is, in this example configuration, arranged inside the tank 3 in such a way that a portion 47 of the second transmission line probe 41 extending from the first probe end 43 to the impedance discontinuity - here provided by the reference reflector 45 - is exclusively located in the second tank portion 15.

Fig 4 schematically illustrates an example configuration of the second radar gauging system 39 comprised in the tank arrangement 1 in fig 3. The second radar gauging system 39 comprises the above-mentioned second transmission line probe 41, a second transceiver 49, and second processing circuitry 51. The first probe end 43 of the second transmission line probe 41 is coupled to the second transceiver 49, which is configured to generate and transmit an electromagnetic transmit signal $S_T$ to the second transmission line probe 41. The second transmission line probe 41 is configured to guide the transmit signal $S_T$ from the first probe end 43 towards and into the substance composition in the tank, and to return to the second transceiver 49 an electromagnetic reflection signal $S_R$ resulting from reflection of the transmit signal $S_T$ at at least one impedance discontinuity encountered thereby. One such impedance discontinuity may be provided by the level of the surface 53 of the substance composition, and another such impedance discontinuity may be provided by the reference reflector 45.

By evaluating a timing relation between the transmit signal $S_T$ and the reflection signal $S_R$, using techniques that are *per se* well-known to the person of ordinary skill in the art, the second processing circuitry 51 may be configured to estimate the distance from the first probe end 43, which is a reference location at an interface between the second transceiver 49 and the second transmission line probe 41 and the surface 53 of the product composition. The second processing circuitry 51 may additionally be configured to estimate the distance between the surface 53 of the product composition and the impedance discontinuitiy provided by the reference reflector 45. By comparing a distance between the surface 53 and the reference reflector 45 thus estimated based on a time-of-flight of the transmit signal $S_T$ (and reflection signal $S_R$) along the portion 47 of the second transmission line probe 41 with the corresponding known second distance between the first probe end 43 and the impedance discontinuity provided by the reference reflector 45, the propagation speed of the transmit signal $S_T$ (and reflection signal $S_R$) can be estimated. From the estimated propagation speed another signal propagation property, such as the (average) dielectric constant, can be estimated, and/or conclusions can be drawn about the material composition through which the transmit signal $S_T$ (and reflection signal $S_R$) has been propagated. It should be noted that the impedance discontinuity may be defined by other means than the reference reflector 45 provided as illustration here. For example, the impedance discontinuity may be provided by a second probe end.

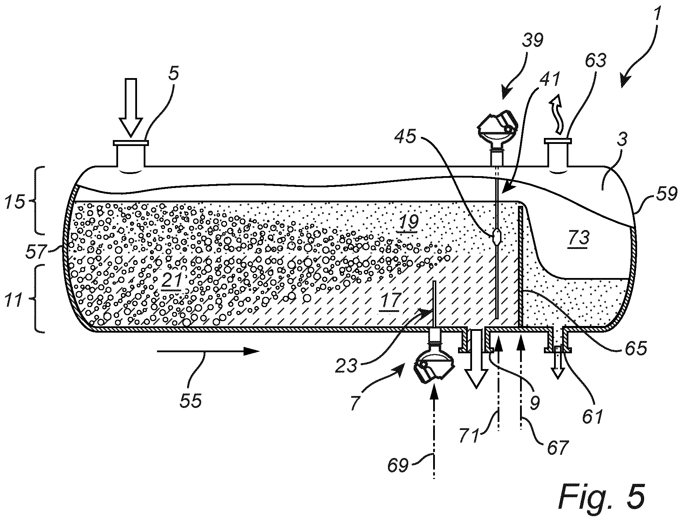

Fig 5 schematically illustrates a third example of a tank arrangement 1 according to embodiments of the present invention, in the form of a phase separator arrangement. The tank 3 is elongated in a substantially horizontal first direction 55, and extends in the first direction 55 between a first tank end 57 and a second tank end 59. As can be seen in fig 5, the tank 3 has an inlet 5, a first outlet 9, a second outlet 61, an optional third outlet 63, and an overflow weir 65.

The inlet 5 is arranged adjacent to the first tank end 57, the overflow weir 65 is arranged at a weir position 67, spaced apart from the inlet 5 in the first direction 55, the first outlet 9 is arranged at a bottom of the tank 3 horizontally between the inlet 5 and the weir position 67, the second outlet 61 is arranged at the bottom of the tank 3 horizontally between the weir position 67 and the second tank end 59, the optional third outlet 63 is arranged at the top of the tank 3, the first radar gauging system 3 is arranged at a first gauging position 69, between the inlet 5 and the weir position 67, and an optional second radar gauging system 39 is arranged at a second gauging position 71, between the inlet 5 and the weir position 67. The first gauging position 69 and the second gauging position 71 may advantageously be arranged close to each other, in the first direction 55.

When the tank arrangement 1 in fig 5 is in use for phase separation, a flow of a substance composition is received through the inlet 5. The substance composition includes at least a liquid first substance 17 having a first density, and a liquid second substance 19 having a second density, lower than the first density. The first substance 17 may, for example, be water, and the second substance 19 may, for example, be oil. When entering the tank 3 through the inlet, the substance composition may be in the form of an emulsion 21 of the first substance 17 and the second substance 19. When progressing through the tank in the horizontal first direction 55 at an appropriate rate, the emulsion 21 is separated into pure first substance 17 at the bottom of the tank 3, with pure second substance 19 floating on top of the first substance 17. Beetween the thus separated layers of first substance 17 and second substance 19, an emulsion boundary layer may remain. First substance 17 is output through the first outlet 9, and second substance 19 is output through the second outlet 61, after having flowed over the overflow weir 65. A third, gaseous substance 73, may be output through the third outlet 63.

As has been described further above, the first radar gauging system 7 may be used for estimating a signal propagation property, such as dielectric constant, of the substance composition within a first volume surrounding the above-defined portion 29 of the first transmission line probe 23, and the second radar gauging system 39 can be used for determining the level of the second substance 19, such as oil, in the tank 3, and/or a signal propagation property, such as dielectric constant, of the substance composition within a second volume surrounding the above-defined portion 47 of the second transmission line probe 41. One or more of these measures may be used for controlling operation of the phase separation provided by the tank arrangement 1 in fig 5, for instance by controlling the flow rate of substance composition at the inlet 5. If the estimated signal propagation property in the first level range surrounding the portion 29 of the first transmission line probe 23 indicates that there is a mixture of first substance 17 and second substance 19 at the bottom of the tank 3 at the first outlet 9, the first oulet 9 may be closed and the flow of substance composition at the inlet 5 stopped to ensure that second substance 19, such as oil, is not discarded to the environment through the first outlet 9.

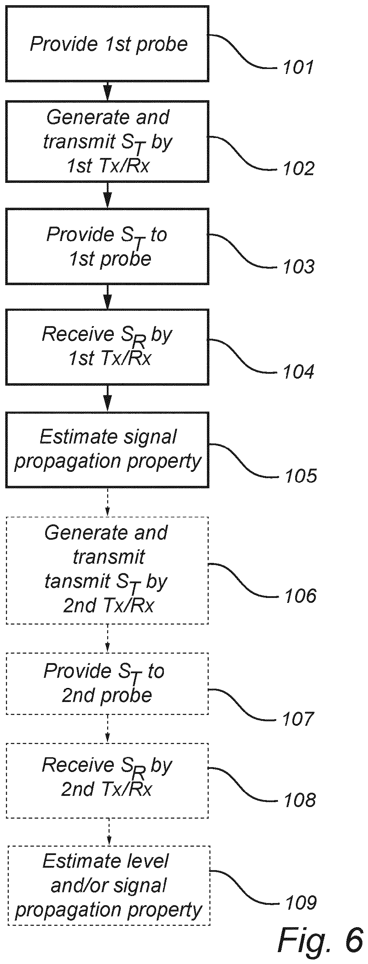

A method according to an example of the present invention will now be described with reference to the flow-chart in fig 6. Other figures may also be referred to as indicated in the text.

Referring to fig 6, there is provided 101 a first transmission line probe 23 having a first probe end 25 and an impedance discontinuity 27 spaced apart along the first transmission line probe from the first probe end by a known first distance, the first transmission line probe 23 being arranged inside the tank 3 in such a way that a portion 29 of the first transmission line probe 23 extending from the first probe end 25 to the impedance discontinuity 27 is exclusively located in a first, relatively low, tank portion 11.

An electromagnetic transmit signal $S_T$ is generated and transmitted 102, by a first transceiver 31. The transmit signal $S_T$ may, for example, be provided in the form of a pulse train and/or as a continuous signal with time-varying frequency, as will be *per* se well-known to one of ordinary skill in the art.

The transmit signal $S_T$ is provided 103 to the first probe end 25 of the first transmission line probe 23.

An electromagnetic reflection signal $S_R$ resulting from reflection of the transmit signal $S_T$ at the impedance discontinuity 27 of the first transmission line probe 23 is received 104 by the first transceiver 31 from the first transmission line probe 23.

Thereafter, a signal propagation property, such as the (average) dielectric constant, of the substance composition within a first volume surrounding the portion 29 of the first transmission line probe is estimated 105 by the first processing circuitry 33, based on a timing relation between the transmit signal $S_T$ and the reflection signal $S_R$ and on a known length of the portion 29 of the first transmission line probe 23.

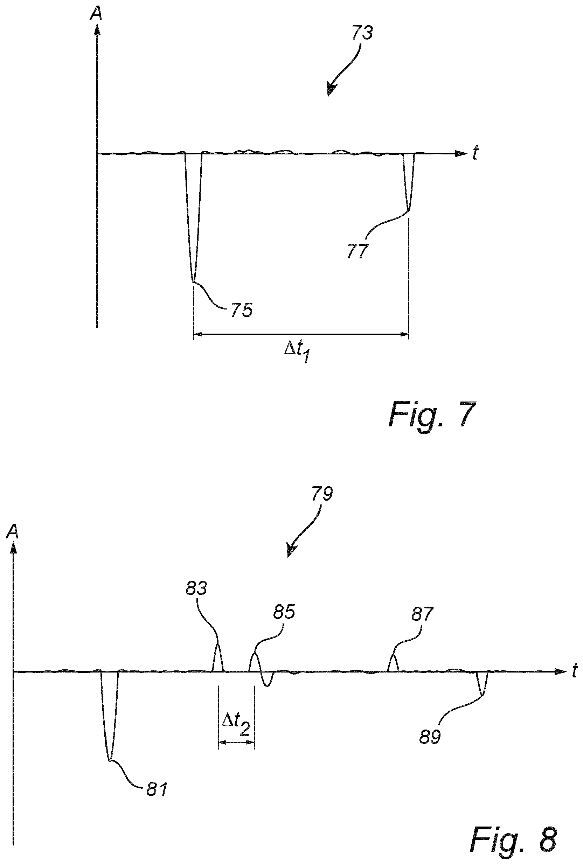

Fig 7 is an exemplary echo curve 73 obtained using the first radar gauging system 7. The echo curve, which illustrates a relation between time-of-flight t for each echo and the amplitude A of the echo signal, may, for example, be obtained using time-expansion techniques that are *per se* well-known in the field of level gauging using pulsed electromagnetic signals. As can be seen in the schematic echo curve 73 in fig 7, there is a first echo peak 75 and a second echo peak 77. The first echo peak 75 results from reflection of the transmit signal $S_T$ at the impedance discontinuity provided by the connection between the first transceiver 31 and the first probe end 25 of the first transmission line probe 23. This echo peak 75 is used as a reference position indicating the position along the first transmission line probe 23 of the first probe end 25, and is sometimes referred to as a fiducial pulse. In the exemplary echo curve 73 in fig 7, the second echo peak 77 results from reflection of the transmit signal $S_T$ at the impedance discontinuity defining the extension along the first transmission line probe 23 of the portion 29 of the first transmission line probe 23. As was mentioned further above, this impedance discontinuity may, for example, be provided by the second probe end of the first transmission line probe 23.

Thus, an "electrical distance" between the first probe end 25 and the impedance discontinuity 27 can be determined based on the difference in time-of-flight $\Delta t_1$ for the first echo peak 75 and the second echo peak 77, respectively. The thus determined electrical distance is a function of the difference in time-of-flight $\Delta t_1$ and the propagation speed of the transmit signal within a first volume of the substance composition surrounding the portion 29 of the first transmission line probe 23. Accordingly, the propagation speed can be estimated based on the electrical distance (or the difference in time-of-flight) and the known length of the portion 29 of the first transmission line probe 23 (the physical distance between the first probe end 25 and the impedance discontinuity 27). From the propagation speed, a signal propagation property (such as the average relative dielectric constant) within the first volume of the substance composition surrounding the portion 29 of the first transmission line probe 23 can be estimated. From the signal propagation property, in turn, conclusions can be drawn about the substance composition in the first tank portion 11. For instance, as in the case of the phase separating tank arrangement 1 in fig 5, it can be determined if the portion 29 of the first transmission line probe 23 is surrounded by pure water, or by an oil-water emulsion and, if so, the emulsion ratio. In applications where the first substance is water or another substance with a very high dielectric constant, it may be beneficial to provide the first transmission line probe 23 with a thin coating of a dielectric material, such as PTFE.

As previously mentioned, the tank arrangement according to embodiments of the invention may comprise a second radar gauging system. One example of such a tank arrangement is schematically shown in fig 3, and another is schematically shown in fig 5. When performed using such a tank arrangement 1, the method according to embodiments of the invention may optionally additionally comprise generating and transmitting 106 an electromagnetic transmit signal $S_T$ by a second transceiver 49, providing 107 the transmit signal $S_T$ to the first probe end 43 of the second transmission line probe 41, and receiving 108, by the second transceiver 49, an electromagnetic reflection signal $S_R$ resulting from reflection of the transmit signal $S_T$ at one or more impedance discontinuities encountered thereby.

Thereafter, the level of the surface 53 of the substance composition in the tank 3 (referring to fig 3) and/or a signal propagation property, such as the (average) dielectric constant, of the substance composition within a second volume surrounding the portion 47 of the second transmission line probe 41 is estimated 109 by the second processing circuitry 51, based on a timing relation between the transmit signal $S_T$ and the reflection signal $S_R$ and on a known length of the portion 47 of the second transmission line probe 41.

Fig 8 is an exemplary echo curve 79 obtained using the second radar gauging system 39. The echo curve, which illustrates a relation between time-of-flight t for each echo and the amplitude A of the echo signal, may, for example, be obtained using time-expansion techniques that are *per se* well-known in the field of level gauging using pulsed electromagnetic signals. As can be seen in the schematic echo curve 79 in fig 8, there is a first echo peak 81, a second echo peak 83, a third echo peak 85, a fourth echo peak 87, and a fifth echo peak 89. The first echo peak 81 results from reflection of the transmit signal $S_T$ at the impedance discontinuity provided by the connection between the second transceiver 49 and the first probe end 43 of the second transmission line probe 41. This echo peak 81 is used as a reference position indicating the position along the second transmission line probe 41 of the first probe end 43, and is sometimes referred to as a fiducial pulse. In the exemplary echo curve 79 in fig 8, the second echo peak 83 results from reflection of the transmit signal $S_T$ at the surface 53 of the substance composition, the third echo peak 85 results from reflection at the reference reflector 45, the fourth echo peak 87 results from reflection at an exemplary substance interface along the second transmission line probe 41, and the fifth echo peak 89 results from reflection at the second probe end of the second transmission line probe 41. As has been mentioned earlier, the reference reflector 45 provides the impedance discontinuity that defines the extension along the second transmission line probe 41 of the portion 47 of the second transmission line probe 41.

Thus, an "electrical distance" between the surface 53 of the substance composition and the impedance discontinuity provided by the reference reflector 45 can be determined based on the difference in time-of-flight $\Delta t_2$ for the second echo peak 83 and the third echo peak 85, respectively. The thus determined electrical distance is a function of the difference in time-of-flight $\Delta t_2$ and the propagation speed of the transmit signal within a second volume of the substance composition surrounding the portion 47 of the second transmission line probe 41. Accordingly, the propagation speed can be estimated based on the electrical distance (or the difference in time-of-flight) and the known length of the portion 47 of the second transmission line probe 41 (the physical distance between the first probe end 43 and the impedance discontinuity provided by the reference reflector 45). This known distance is equivalent to the position along the second transmission line probe 41 of the reference reflector 45. From the propagation speed, a signal propagation property (the average relative dielectric constant) within the second volume of the substance composition surrounding the portion 47 of the second transmission line probe 41 can be estimated. From the signal propagation property, in turn, conclusions can be drawn about the substance composition in the second tank portion 15. For instance, as in the case of the phase separating tank arrangement 1 in fig 5, it can be determined if the portion 47 of the second transmission line probe 41 is (partly) surrounded by pure oil, or by an oil-water emulsion and, if so, the emulsion ratio. This may be used for further improved control of operation of the tank arrangement 1.

The person skilled in the art realizes that the present invention by no means is limited to the preferred embodiments described above. For example, other probe arrangements may be feasible.

The "substances" are not, in general, limited to any particular material phase, and may thus include solids, liquids and gases. Some examples of substances can be various petroleum products, water, sludge or sand etc.

The "transceiver" may be one functional unit capable of transmitting and receiving electromagnetic signals, or may be a system comprising separate transmitter and receiver units.

The tank may be any container or vessel capable of containing a product, and may be metallic, or partly or completely non-metallic, open, semi-open, or closed.

**Claims**

1. A tank arrangement, comprising:

   a tank having a first tank portion defined by a first range of vertical distances from a bottom of the tank, and a second tank portion defined by a second range of vertical distances from the bottom of the tank, the second range of vertical distances only containing distances greater than all distances in the first range of vertical distances;
   an inlet for receiving a substance composition including at least a liquid first substance having a first density and a liquid second substance having a second density lower than the first density;
   at least a first radar gauging system arranged at the tank, the first radar gauging system comprising:

      a first transceiver for generating, transmitting and receiving electromagnetic signals;
      a first transmission line probe having a first probe end coupled to the first transceiver and an impedance discontinuity spaced apart along the first transmission line probe from the first probe end by a known first distance, the first transmission line probe being arranged inside the tank in such a way that a portion of the first transmission line probe extending from the first probe end to the impedance discontinuity is exclusively located in the first tank portion, the first transmission line probe being configured to guide an electromagnetic transmit signal provided by the first transceiver from the first probe end to the impedance discontinuity, and to return to the first transceiver an electromagnetic reflection signal resulting from reflection of the transmit signal at the impedance discontinuity; and
first processing circuitry coupled to the first transceiver and configured to estimate, based on a timing relation between the transmit signal and the reflection signal and on the known first distance, a signal propagation property of the substance composition within a first volume surrounding the portion of the first transmission line probe.

2. The tank arrangement according to claim 1, the inlet being arranged in the second tank portion.

3. The tank arrangement according to claim 1 or 2, comprising an outlet arranged in the first tank portion for output of first substance separated from the substance composition.

4. The tank arrangement according to any one of the preceding claims, the first processing circuitry of the first radar gauging system being configured to determine, based on the estimated signal propagation property, a known signal propagation property of the first substance, and a known signal propagation property of the second substance, a measure indicative of a proportion of the first substance in the substance composition within the first volume surrounding the portion of the first transmission line probe.

5. The tank arrangement according to any one of the preceding claims, the impedance discontinuity of the first transmission line probe of the first radar gauging system being at a higher vertical level than the first probe end.

6. The tank arrangement according to any one of the preceding claims, the impedance discontinuity of the first transmission line probe of the first radar gauging system being constituted by a second probe end of the first transmission line probe.

7. The tank arrangement according to any one of the preceding claims, the first transmission line probe of the first radar gauging system comprising:

   a probe conductor coupled to the transceiver; and
   a dielectric layer at least partly enclosing the probe conductor.

8. The tank arrangement according to claim 7, the dielectric layer fully enclosing the probe conductor in the probe portion.

9. The tank arrangement according to any one of the preceding claims,

   the tank being elongated in a substantially horizontal first direction, and extending in the first direction between a first tank end and a second tank end;
   the inlet being adjacent to the first tank end;
   the tank comprising an overflow weir arranged at a weir position between the inlet and the second tank end;
   the tank comprising a first outlet arranged at a bottom of the tank horizontally between the inlet and the weir position, for output of first substance separated from the substance composition;
   the tank comprising a second outlet arranged at a bottom of the tank horizontally between the weir position and the second tank end, for output of second substance separated from the substance composition; and
   the first radar gauging system being arranged to gauge the substance composition at a first gauging position between the inlet and the weir position.

10. The tank arrangement according to claim 9, wherein the first radar gauging system is arranged horizontally between the first outlet and the weir position.

11. The tank arrangement according to any one of the preceding claims, comprising:
    a second radar gauging system arranged at the tank, the second radar gauging system comprising:

    a second transceiver for generating, transmitting and receiving electromagnetic signals;
    a second transmission line probe having a first probe end coupled to the second transceiver, the second transmission line probe being configured to guide an electromagnetic transmit signal from the second transceiver from the first probe end towards the substance composition in the tank, and to return to the second transceiver an electromagnetic reflection signal resulting from reflection of the transmit signal at a surface of the substance composition; and
    second processing circuitry coupled to the second transceiver and configured to estimate, based on a timing relation between the transmit signal and the reflection signal, a level of the surface of the substance composition.

12. The tank arrangement according to claim 11, the second transmission line probe having an impedance discontinuity spaced apart along the second transmission line probe from the first probe end by a known second distance, the second transmission line probe being arranged inside the tank in such a way that a portion of the second transmission line probe extending from the first probe end to the impedance discontinuity is exclusively located in the second tank portion; and
the second processing circuitry being configured to estimate, based on the timing relation between the transmit signal and the reflection signal and on the known second distance, a signal propagation property of the substance composition within a second volume surrounding the portion of the second transmission line probe.

13. A method of estimating a signal propagation property of a substance composition in a first tank portion defined by a first range of vertical distances from a bottom of the tank, the first tank portion being below a second tank portion defined by a second range of vertical distances from the bottom of the tank, the method comprising:

   providing a first transmission line probe having a first probe end and an impedance discontinuity spaced apart along the first transmission line probe from the first probe end by a known first distance, the first transmission line probe being arranged inside the tank in such a way that a portion of the first transmission line probe extending from the first probe end to the impedance discontinuity is exclusively located in the first tank portion;
   generating and transmitting, by a first transceiver, an electromagnetic transmit signal;
   providing the transmit signal to the first probe end of the first transmission line probe;
   receiving, by the first transceiver from the first transmission line probe, an electromagnetic reflection signal resulting from reflection of the transmit signal at the impedance discontinuity of the first transmission line probe; and
   estimating, by first processing circuitry coupled to the first transceiver, the signal propagation property of the substance composition within a first volume surrounding the portion of the first transmission line probe, based on a timing relation between the transmit signal and the reflection signal, and on the known first distance.

14. The method according to claim 13, comprising:
determining, by the first processing circuitry, a measure indicative of a proportion of the first substance in the substance composition within the first volume surrounding the portion of the first transmission line probe, based on the estimated signal propagation property, a known signal propagation property of the first substance, and a known signal propagation property of the second substance.

15. The method according to claim 13 or 14, comprising:

providing a second transmission line probe having a first probe end and an impedance discontinuity spaced apart along the second transmission line probe from the first probe end by a known second distance, the second transmission line probe being arranged inside the tank in such a way that a portion of the second transmission line probe extending from the first probe end to the impedance discontinuity is exclusively located in the second tank portion;
generating and transmitting, by a second transceiver, an electromagnetic transmit signal;
providing the transmit signal to the first probe end of the second transmission line probe;
receiving, by the second transceiver from the second transmission line probe, an electromagnetic reflection signal resulting from reflection of the transmit signal at the impedance discontinuity of the second transmission line probe; and
estimating, by first processing circuitry coupled to the first transceiver, the signal propagation property of the substance composition within a first volume surrounding the portion of the first transmission line probe, based on a timing relation between the transmit signal and the reflection signal, and on the known second distance.

Fig. 1

Fig. 2A

Fig. 2B

*Fig. 3*

*Fig. 4*

*Fig. 5*

```
┌─────────────────────┐
│   Provide 1st probe │ ⟋ 101
└─────────────────────┘
           │
           ▼
┌─────────────────────┐
│   Generate and      │
│   transmit S_T by   │ ⟋ 102
│   1st Tx/Rx         │
└─────────────────────┘
           │
           ▼
┌─────────────────────┐
│   Provide S_T to    │
│   1st probe         │ ⟋ 103
└─────────────────────┘
           │
           ▼
┌─────────────────────┐
│   Receive S_R by    │
│   1st Tx/Rx         │ ⟋ 104
└─────────────────────┘
           │
           ▼
┌─────────────────────┐
│   Estimate signal   │
│ propagation property│ ⟋ 105
└─────────────────────┘
           ┊
           ▼
┌ ─ ─ ─ ─ ─ ─ ─ ─ ─ ─ ┐
│   Generate and      │
│   transmit          │
│   tansmit S_T by    │ ⟋ 106
│   2nd Tx/Rx         │
└ ─ ─ ─ ─ ─ ─ ─ ─ ─ ─ ┘
           ┊
           ▼
┌ ─ ─ ─ ─ ─ ─ ─ ─ ─ ─ ┐
│   Provide S_T to    │
│   2nd probe         │ ⟋ 107
└ ─ ─ ─ ─ ─ ─ ─ ─ ─ ─ ┘
           ┊
           ▼
┌ ─ ─ ─ ─ ─ ─ ─ ─ ─ ─ ┐
│   Receive S_R by    │
│   2nd Tx/Rx         │ ⟋ 108
└ ─ ─ ─ ─ ─ ─ ─ ─ ─ ─ ┘
           ┊
           ▼
┌ ─ ─ ─ ─ ─ ─ ─ ─ ─ ─ ┐
│   Estimate level    │
│   and/or signal     │ ⟋ 109
│ propagation property│
└ ─ ─ ─ ─ ─ ─ ─ ─ ─ ─ ┘
```

*Fig. 6*

Fig. 7

Fig. 8

**EP 4 521 077 A1**

Europäisches Patentamt

European Patent Office

Office européen des brevets

# EUROPEAN SEARCH REPORT

Application Number

EP 23 19 6250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 828 930 B2 (SAAB ROSEMOUNT TANK RADAR AB [SE]) 7 December 2004 (2004-12-07) <br> * figure 1 * <br> * column 3, line 8 – line 15 * <br> ----- | 1,2,4-8, 11-15 | INV. <br> G01F23/284 <br> G01S13/88 |
| X <br> A | US 2019/219531 A1 (JOHNSON BRIAN [GB] ET AL) 18 July 2019 (2019-07-18) <br> * figure 2 * <br> * paragraph [0025] * <br> ----- | 1,3,9, 10,13 <br> 11,12,15 | |
| X <br> A | US 9 939 308 B2 (GRIESHABER VEGA KG [DE]) 10 April 2018 (2018-04-10) <br> * figure 1 * <br> * column 4, line 6 – line 42 * <br> ----- | 1,13 <br> 11,12,15 | |
| A | US 10 184 820 B2 (ROSEMOUNT TANK RADAR AB [SE]) 22 January 2019 (2019-01-22) <br> * figures 2A, 5A * <br> ----- | 1-15 | |
| | | | TECHNICAL FIELDS SEARCHED (IPC) <br><br> G01F <br> G01S |

The present search report has been drawn up for all claims

| Place of search | Date of completion of the search | Examiner |
|---|---|---|
| The Hague | 9 February 2024 | Régert, Tamás |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P04C01)

16

## ANNEX TO THE EUROPEAN SEARCH REPORT
## ON EUROPEAN PATENT APPLICATION NO.

EP 23 19 6250

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2024

| Patent document cited in search report | | Publication date | Patent family member(s) | | Publication date |
|---|---|---|---|---|---|
| US 6828930 | B2 | 07-12-2004 | NONE | | |
| US 2019219531 | A1 | 18-07-2019 | US | 2015362447 A1 | 17-12-2015 |
| | | | US | 2019219531 A1 | 18-07-2019 |
| US 9939308 | B2 | 10-04-2018 | CN | 105841773 A | 10-08-2016 |
| | | | EP | 3054271 A1 | 10-08-2016 |
| | | | HU | E036364 T2 | 30-07-2018 |
| | | | TW | 201640082 A | 16-11-2016 |
| | | | US | 2016223381 A1 | 04-08-2016 |
| US 10184820 | B2 | 22-01-2019 | CN | 107884035 A | 06-04-2018 |
| | | | CN | 206556721 U | 13-10-2017 |
| | | | EP | 3301413 A1 | 04-04-2018 |
| | | | US | 2018094962 A1 | 05-04-2018 |

EPO FORM P0459

For more details about this annex : see Official Journal of the European Patent Office, No. 12/82